Europäisches Patentamt

European Patent Office

Office européen des brevets

(11) Veröffentlichungsnummer : **0 046 518**
**B1**

# (12) EUROPÄISCHE PATENTSCHRIFT

(45) Veröffentlichungstag der Patentschrift :
**13.07.83**

(21) Anmeldenummer : **81106015.1**

(22) Anmeldetag : **31.07.81**

(51) Int. Cl.³ : **A 61 B 17/06, B 65 D 75/58**.

(54) Packung für chirurgisches Nahtmaterial.

(30) Priorität : **26.08.80 DE 3032037**

(43) Veröffentlichungstag der Anmeldung :
**03.03.82 Patentblatt 82/09**

(45) Bekanntmachung des Hinweises auf die Patenterteilung : **13.07.83 Patentblatt 83/28**

(84) Benannte Vertragsstaaten :
**AT BE CH DE FR IT LI LU NL SE**

(56) Entgegenhaltungen :
**DE A 1 966 391**
**FR A 2 188 569**
**US A 3 174 654**
**US A 3 389 850**
**US A 4 089 410**
**US A 4 135 622**

(73) Patentinhaber : **Intermedicat GmbH**
**Gerliswilstrasse 45**
**CH-6020 Emmenbrücke (CH)**

(72) Erfinder : **Fontana, Karl-Heinz**
**Ahornweg 3**
**D-3501 Körle (DE)**

(74) Vertreter : **von Kreisler, Alek, Dipl.-Chem. et al**
**Deichmannhaus am Hauptbahnhof**
**D-5000 Köln 1 (DE)**

Packung für chirurgisches Nahtmaterial

Die Erfindung betrifft eine Packung für chirurgisches Nahtmaterial, mit zwei an den Rändern gegeneinandergelegten und abdichtend miteinander verbundenen, das Nahtmaterial einschließenden Folienteilen, die an einem Rand Aufreißlaschen aufweisen, und mit einer zwischen den Folienteilen angeordneten, die wesentliche Menge des Nahtmaterials enthaltenden Faltkarte, aus der ein Fadenende des Nahtmaterials herausragt.

In den bekannten Nahtmaterialpackungen ist das chirurgische Nahtmaterial, z. B. ein Faden, an dem auch eine Nadel angebracht sein kann, keimdicht abgeschlossen zwischen zwei Folienteilen untergebracht. Die Packung enthält in der Regel einen Fadenträger sowie Etiketten und Instruktionsmaterial. Wird die Packung im Operationssaal geöffnet, dann liegen der Fadenträger und die anderen in der Packung enthaltenen Zettel und Materialien frei, um aus der Packung entnommen werden zu können.

Das Operationspersonal muß also die Packung öffnen und die in ihr enthaltenen Gegenstände entnehmen, wobei anschließend noch das Nahtmaterial von dem Fadenträger abgenommen werden muß. Die leere Packung sowie die Etiketten u. dgl. werden fortgeworfen.

Es ist eine Packung für chirurgisches Nahtmaterial bekannt (US-PS-3 939 969), bei der zwischen zwei miteinander verschweißten Folienteilen eine Faltkarte angeordnet ist, die das Nahtmaterial enthält. Die Folienteile weisen an einem Ende Aufreißlaschen auf, die gemeinsam seitlich abgezogen werden, wobei die Folienteile aufreißen und das eine Ende der Faltkarte freigelegt wird. Aus diesem freigelegten Ende der Faltkarte kann das Nahtmaterial abgezogen werden. Die Faltkarte weist einen Schlitz auf, in den eine an dem freien Ende des Nahtmaterials befestigte gebogene Nadel von außen eingesetzt werden kann. Die Faltkarte ist über eine Klebestelle mit einem der Folienteile verbunden. Bei einer derartigen Packung wird nach dem Aufreißen nur ein kleiner Teil der Faltkarte freigelegt. Wenn sich in der Packung noch Etiketten oder Instruktionsmaterial befindet, kann dieses nur schwer herausgezogen werden, da beim Aufreißen ein großer Teil der Packung geschlossen und für die Entnahme von Gegenständen unzugänglich bleibt. Auch das Entnehmen des Fadenendes kann Schwierigkeiten bereiten, wenn dieses Fadenende sich nach dem Aufreißen in dem noch unversehrten Teil der Packung befindet.

Bekannt ist ferner eine sterile Fadenpackung (DE-GM 71 22 075), bei der zwischen zwei Folienteilen, deren Ränder miteinander versiegelt sind, sich ein Nahtmaterialträger in Form einer einfachen Karte befindet, auf die der Faden aufgewickelt ist. Die Karte ist an die Innenwand der Packung angeklebt, so daß nach dem Öffnen der Packung die Folienteile mit der Karte verbunden bleiben. Hierbei kann das Nahtmaterial aber nur sehr schwer entnommen werden, weil nach dem Aufreißen der Packung der gesamte Nahtmaterialträger noch in der an einer Seite offenen Packung verbleibt, so daß zum Abwickeln des Fadens in den noch unversehrten Teil der Packung eingegriffen werden muß, was in der Regel nur mit einer Pinzette möglich ist.

Weiterhin ist eine Packung für chirurgisches Nahtmaterial bekannt (US-PS 3 319 782), bei der ein wannenförmiges Folienteil durch ein dünnes Folienteil als Deckel abgedeckt ist. In dem umschlossenen Raum befindet sich ein Nahtmaterialträger in Form einer Hülse, in der der Faden in aufgewickelter Form angeordnet ist, wobei der Faden aus dem einen Ende der Hülse herausragt. Die beiden Folien weisen in der Nähe des einen Endes der länglichen Packung eine Aufreißlinie auf. Nach dem Aufreißen besteht die Packung aus einem an einem Ende offenen schlauchförmigen Teil, aus dem heraus eine Schleife des aufgewickelten Fadens vorsteht. Die den Faden enthaltende Hülse ist an dem der Aufreißstelle abgewandten Ende durch eine Lasche mit den Folienteilen verbunden. Beim Aufreißen der Packung wird das abgerissene Ende von den übrigen Teilen der Packung getrennt, so daß es ein separates Teil darstellt, das im Operationssaal eine Behinderung bilden kann. Das Ergreifen des Fadenendes ist sehr schwierig.

Die bekannten Packungen für Nahtmaterial haben sämtlich den Nachteil, daß beim Aufreißen der Packung nur ein kleiner Teil des Nahtmaterialträgers freigelegt wird, und daß es daher sehr schwierig sein kann, das Ende des Fadens zu ergreifen. Ein weiterer Nachteil besteht darin, daß das abgerissene Teil vollständig von der Packung getrennt wird und eine Behinderung darstellen kann.

Der Erfindung liegt die Aufgabe zugrunde, eine Packung für chirurgisches Nahtmaterial zu schaffen, bei der sämtliche Teile auch nach dem Aufreißen noch einen gewissen Zusammenhalt haben, so daß sich keine lose herumfliegenden Teile ergeben und bei der beim Aufreißen ein großer Teil der den Faden enthaltenden Faltkarte freigelegt wird, so daß der Faden ohne Schwierigkeiten ergriffen werden kann.

Zur Lösung dieser Aufgabe ist erfindungsgemäß vorgesehen, daß das eine Folienteil derart ausgebildet ist, daß sich beim Auseinanderziehen der Aufreißlaschen Reißlinien ergeben, die im wesentlichen parallel zu den Stirnseiten der Packung verlaufen, wobei längs der Stirnseiten dieses Folienteils Streifen stehenbleiben, die zwei einander gegenüberliegende Ränder der Faltkarte überdecken.

Beim Abreißen des einen Folienteiles, das den Deckel bildet, wird die gesamte Oberfläche der Faltkarte freigelegt, mit Ausnahme der Randbereiche der Faltkarte. Diese Randbereiche werden unter den stehengebliebenen seitlichen Streifen des ersten Folienteiles niedergehalten, so daß die Faltkarte nicht aus dem anderen Folienteil herausfallen kann. Beide Folienteile bleiben auch nach dem Aufreißen miteinander verbunden. Das auf der Oberseite der Faltkarte befindliche Fadenende kann ohne Schwierigkeiten ergriffen werden, weil fast die ganze Oberseite der Faltkarte freiliegt und zugänglich ist.

In vorteilhafter Weiterbildung der Erfindung weist die Faltkarte mindestens zwei übereinandergefaltete Flächen, zwischen denen sich die wesentliche Menge des Nahtmaterials befindet, und eine Öffnung auf, durch die hindurch das Nahtmaterial herausziehbar ist. Die Faltkarte bietet die Möglichkeit, eine Bedruckung anzubringen, so daß keine zusätzlichen Etiketten erforderlich sind. Die Öffnung befindet sich vorzugsweise an der den Aufreißlaschen zugewandten Faltkante der Faltkarte. Dies hat den Vorteil, daß das aus der Öffnung herausragende Fadenende auch mit Handschuhen leicht ergriffen und abgezogen werden kann.

Vorzugsweise ist die Faltkarte über eine flexible Lasche mit dem einen Folienteil verbunden. Hierdurch wird erreicht, daß selbst in dem Fall, daß die Faltkarte unter den stehengebliebenen Streifen des ersten Folienteiles herausgleitet, die Verbindung zwischen der Packung und der Faltkarte erhalten bleibt.

Um eine möglichst große Bewegungsfreiheit der Faltkarte in bezug auf die Packung zu erhalten, ist in vorteilhafter Weiterbildung der Erfindung vorgesehen, daß die Lasche mehrere querverlaufende Faltlinien mit einander abwechselnden Faltrichtungen aufweist. Im geschlossenen Zustand der Packung sind die einzelnen durch Faltlinien miteinander verbundenen Abschnitte der Lasche aufeinanderliegend angeordnet. Nach dem Öffnen der Packung wird die Lasche automatisch auseinandergezogen und die Faltung hochgestellt, während der Fadenträger in der Packung verbleibt. Durch das Hochstellen der Faltung entsteht ein Freiraum zwischen Faden und der Oberfläche des Fadenträgers. Dadurch ist ein leichtes Ergreifen des an der Faltstelle eingeklemmten Nahtmaterialendes mit Handschuhen oder einem Nadelhalter möglich.

Um erforderlichenfalls den Fadenträger von der Packung abziehen zu können, ist zweckmäßigerweise mindestens eine der Faltlinien als perforierte Abreißlinie ausgebildet.

Wenn das Nahtmaterial aus einem Faden besteht, der gegebenenfalls auch fest mit einer entsprechenden Nadel verbunden sein kann, ist es zweckmäßig, das Fadenende bzw. die Nadel griffbereit festzulegen. Um dies zu erreichen, weist bei einer bevorzugten Ausführungsform der Erfindung die Lasche einen eine Faltlinie kreuzenden Schlitz zum Einklemmen des Endes des Nahtmaterials bzw. der Nadel auf.

Die den Fadenträger bildende Faltkarte sollte zweckmäßigerweise so ausgebildet sein, daß sie ein flaches Behältnis bildet, in dem sich die wesentliche Menge des Fadens befindet. Bei einer bevorzugten Ausführungsform weist die Faltkarte drei im wesentlichen gleich große, entlang ihrer Längskanten durch Faltlinien verbundene Flächen auf, von denen an der freien Längskante einer der äußeren Flächen die Lasche angeordnet ist. Die Faltkarte bleibt beim normalen Gebrauch geschlossen, wobei das Nahtmaterial aus der Öffnung herausgezogen wird. Um das unbeabsichtigte Auseinanderklappen der Faltkarte zu verhindern, kann die Faltkarte in der Nähe ihrer der Lasche zugewandten Kante Einsteckschlitze für zwei Ecken der gefalteten Lasche aufweisen. Die Lasche trägt dann dazu bei, die Faltkarte geschlossen zu halten.

Die Lasche sollte an einem der Folienteile so angebracht sein, daß sie beim Auseinanderziehen der Folienteile an dem betreffenden Folienteil verbleibt und gleichzeitig auseinandergespreizt wird.

Gemäß einer bevorzugten Ausführungsform der Erfindung ist das zweite Folienteil ein relativ steifer wannenförmiger Behälter, der mit dem ersten Folienteil als Deckel entlang der gegeneinanderliegenden Randbereiche verschlossen ist. Die Lasche des Fadenträgers ist an der Unterseite des Deckels befestigt.

Im folgenden wird unter Bezugnahme auf die Figuren ein Ausführungsbeispiel der Erfindung näher erläutert :

Es zeigen :

Figur 1 eine perspektivische Ansicht der Packung,

Figur 2 einen Schnitt. entlang der Linie II-II der Figur 1,

Figur 3 eine Seitenansicht der Packung beim Hochziehen des Deckels,

Figur 4 eine perspektivische Ansicht der Packung bei hochgeklapptem Deckel vor dem Versiegeln der Folienteile und

Figur 5 den Fadenträger im auseinandergeklappten Zustand, losgelöst von der Packung.

Die dargestellte Packung 10 besteht aus zwei Folienteilen. Das untere Teil der Packung ist ein wannenförmiger, relativ steifer tiefgezogener Behälter 11, der eine rechteckige Vertiefung 11' zur Aufnahme des Nahtmaterialträgers aufweist, und der entlang seiner umlaufenden Kanten Flansche 13 aufweist. Das obere Folienteil ist eine glatte biegsame Folie, die den Deckel 12 bildet und flach an den Flanschen 13 des Behälters 11 anliegt.

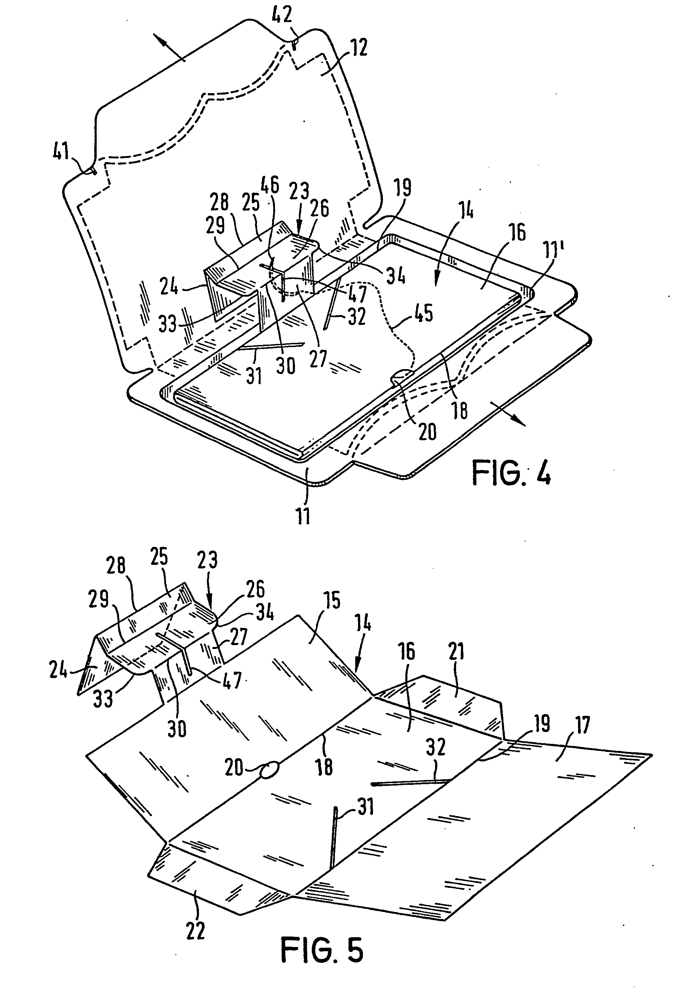

Im Innern des Behälters ist der Fadenträger in Form einer Faltkarte 14 aus Papier angeordnet. Die Faltkarte 14 besteht gemäß Figur 5 aus einem Bogen Papier, der durch Faltlinien 18, 19 in etwa drei gleich große Bereiche 15, 16, 17 unterteilt ist. Etwa in der Mitte der Faltlinie 18 befindet sich eine runde Öffnung 20 zum Hindurchstecken des Fadens. Der Faden wird auf den mittleren Bereich 16 der geöffneten Faltkante aufgelegt und sein Ende durch die Öffnung 20 hindurchgesteckt. Um zu verhindern, daß der Faden aus den Stirnseiten der Faltkarte herausgleitet, sind an den stirnseitigen Enden des mittleren Bereichs 16 umklappbare Lappen 21, 22 vorgesehen. Diese Lappen werden um 180° herumgeklappt. Anschließend wird der äußere Bereich 17 um die Faltlinie 19 geklappt, so daß er die Lappen 21, 22 und den mittleren Bereich 16 überdeckt. Schließlich wird der Bereich 15 über den umgeklappten Bereich 17 gefaltet. Die Rückseite des Bereichs 15 bildet nun die Unterseite und die Rückseite des Bereichs 16 die Oberseite des zusammengefalteten Fadenträgers (Figur 4).

Von der freien Längskante des Bereichs 15 steht die Lasche 23 ab. Der Endlappen 24 der Lasche 23 ist an der Unterseite des Deckels 12 angeschweißt oder angeklebt, und zwar in demjenigen Bereich, der beim Hochziehen des Deckels 12 gemäß Figur 4 von dem Behälter 11 abgehoben wird. Die Lasche 23 besteht außer dem Lappen 24 aus den Bereichen 25, 26 und 27, die jeweils durch Knicklinien 28, 29 bzw. 30 voneinander getrennt sind. Die Knicklinien 28, 29 und 30, die sämtlich parallel zueinander und in Längsrichtung der Faltkarte 14 verlaufen, haben abwechselnd einander entgegensetzte Knickrichtungen, so daß die Lasche 23 bei geschlossenem Deckel 12 ziehharmonikaartig zusammengefaltet ist. Entlang der Knicklinien 28 und 30 sind Perforationen vorgesehen, um gewünschtenfalls ein definiertes Abreißen des Fadenträgers 14 vom Deckel 12 zu ermöglichen.

An der der Öffnung 20 gegenüberliegenden Kante des Bereichs 16 der Faltkarte 14 sind, wie die Figuren 4 und 5 zeigen, geradlinige Einschnitte 31, 32 vorgesehen, die in Richtung auf die Längsmittelachse des Bereichs 16 V-formig konvergieren, aber nicht zusammenkommen. Die Schlitze 31, 32 dienen zum Einstecken der Ecken 33, 34 des Abschnitts 26 der Lasche 23. Der sich an den Bereich 15 anschließende Abschnitt 27 der Lasche 23 hat eine geringere Breite als der nächstfolgende Abschnitt 26, so daß die Ecken 33, 34 entstehen, die, wenn der Abschnitt 27 der Lasche über den Bereich 16 des Fadenträgers gefaltet wird, in die Schlitze 31, 32 gesteckt werden können, so daß die Lasche gleichzeitig das Verschlußteil für die zusammengefaltete Faltkarte bildet.

Der Nähgutfaden 45, dessen Anfang gemäß Figur 4 durch die Öffnung 20 der Faltkarte herausragt, weist an seinem Ende eine metallische Nadel 46 auf. Die Festlegung dieser Nadel erfolgt in einem Schlitz 47, in den Abschnitten 26 und 27 der Lasche 23. Da diese Abschnitte 26 und 27 sich beim Anheben des Deckels 12 von der Faltkarte 14 abheben, wird die Nadel 46 mit angehoben, so daß sie sich dem Benutzer leicht greifbar präsentiert. Wie aus Figur 4 hervorgeht, bildet der Abschnitt 27 bei angehobenem Deckel 12 den vertikalen und der Abschnitt 26 den horizontalen Bereich einer Stufe.

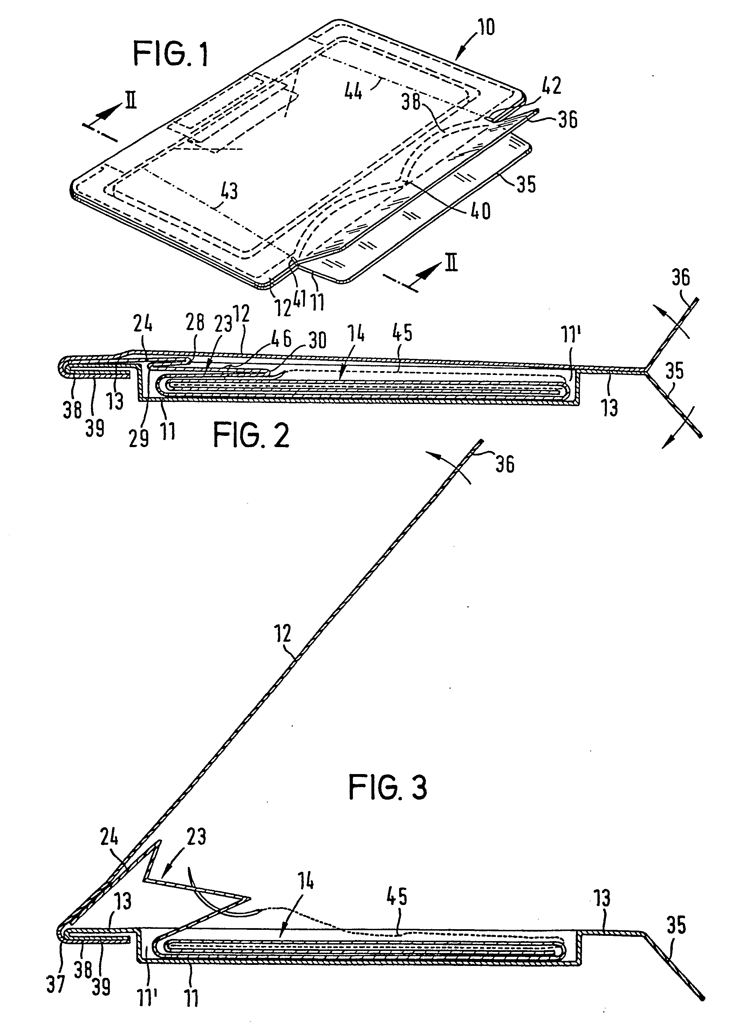

An der der Lasche 23 gegenüberliegenden Längsseite der Packung 10 befinden sich an dem Behälter 11 bzw. dem Deckel 12 Lappen 35, 36, die gemäß Figur 2 auseinandergezogen werden können, um die Packung zu öffnen. Im geöffneten Zustand, der in Figur 3 dargestellt ist, sind entlang der den Lappen 35, 36 abgewandten Längskante 37 der Behälter 11 und der Deckel 12 immer noch miteinander verbunden. Die Schweißnaht 38, die die Folienteile 11, 12 miteinander verbindet, verläuft außerhalb des Bereichs, an dem die Lasche 23 am Deckel 12 befestigt ist. Der Bereich 39, in dem sich die Schweißnaht 38 entlang derjenigen Längskante befindet, die der Lasche 23 benachbart ist, ist gemäß Figur 3 um 180° umgebogen, so daß der Bereich der Schweißnaht 38 dort unter den Flansch 13 des Behälters 11 gebogen ist. Diese Umbiegung hat den Vorteil, daß die Abmessungen der Packung. verringert werden. Außerdem wird verhindert, daß beim Aufreißen des Deckels 12 der gesamte Deckel 12 von dem Behälter 11 abgetrennt wird.

In gleicher Weise können zur Verringerung der breiten Abmessungen der Packung auch die Lappen 35 und 36, die nicht durch eine Schweißnaht unmittelbar miteinander verbunden sind, umgefaltet werden. Die Lappen 35, 36 sind vorzugsweise unterschiedlich lang ausgeführt, so daß sie leicht ergriffen und auseinandergezogen werden können.

Parallel zu den Lappen 35 und 36 verläuft die Schweißnaht 38, wie Figur 1 zeigt, in Form zweier aneinandergesetzter Bögen, die in Höhe der Mitte der Lappen 35 und 36 gegeneinanderstoßen und eine Spitze 40 bilden. Die Bögen sind jeweils von den Laschen 35, 36 zum Packungsinneren hin ausgebaucht und ihre von der Spitze 40 abgewandten Enden führen jeweils zu einem Fußpunkt der Lappen 35 und 36, d. h. an einen Punkt, an dem die Lappen, die kürzer sind als die Packung, von der im wesentlichen rechteckigen Kontur der Packung abstehen. An diesen Fußpunkten, an denen die Lappen 35, 36 in die Längskante der Packung 10 übergehen, befinden sich Einschnitte 41, 42 in den beiden Folienteilen 11, 12, oder mindestens in dem Deckel 12. Diese Einschnitte 41 und 42 erstrecken sich ein Stück quer durch die Siegelnaht 38 hindurch. Sie bewirken, daß beim Auseinanderziehen der Laschen 35, 36 das Deckelteil 12 an den Schlitzen 41, 42 zerreißt und daß die Reißlinien 43, 44 sich gemäß Figur 1 im wesentlichen parallel zu den Stirnseiten der Verpackung bis zur gegenüberliegenden Längsseite fortsetzen. Auf diese Weise bleiben längs der Stirnseiten der Packung 10 Streifen des Deckelteiles 12 stehen, die die Faltkarte 14 teilweise überdecken und verhindern, daß die Faltkarte 14 bei aufgerissenem Deckel aus der Packung herausfällt. Gleichwohl wird die Lasche 23, die sich etwa in der Mitte des hochgezogenen Deckelteiles befindet, mit hochgezogen und gemäß Figur 4 aufgefaltet, so daß das Ende des Nahtmaterials zum Ergreifen angeboten wird.

Bei der Darstellung gemäß Figur 4 ist der Deckel 12 in seiner Gesamtheit hochgeklappt dargestellt. Dies entspricht dem Zustand vor dem Herstellen der Siegelnaht. Nach dem Aufreißen des Deckels 12 wird dagegen die Faltkarte 14 von den am Behälter 11 verbleibenden Deckelteilen 12 in ihrer Stellung festgehalten, so daß sie nicht aus der Vertiefung 11' herausfällt.

Die Folienteile 11 und 12 bestehen vorzugsweise aus durchsichtigem oder durchscheinendem Material, das bedruckt werden kann. Ein weiterer Vorteil besteht darin, daß die Packung resterilisierbar ist. Das Einlegen des Fadens in die Faltkarte 14 kann vollautomatisch oder halbautomatisch mit entsprechenden Maschinen durchgeführt werden. Der Faden kann dabei mit einer Einschußvorrichtung in das Karteninnere eingebracht, und das Fadenende bzw. die Nadel in den Schlitz 37 eingesteckt werden.

Das Ansiegeln der Lasche 23 an den Deckel 12 erfolgt gleichzeitig mit der Herstellung der Schweißnaht 38.

## Ansprüche

1. Packung für chirurgisches Nahtmaterial, mit zwei an den Rändern gegeneinandergelegten und abdichtend miteinander verbundenen, das Nahtmaterial einschließenden Folienteilen, die an einem Rand Aufreißlaschen aufweisen, und mit einer zwischen den Folienteilen angeordneten, die wesentliche Menge des Nahtmaterials enthaltenden Faltkarte, aus der ein Fadenende des Nahtmaterials herausragt, dadurch gekennzeichnet, daß das eine Folienteil (12) derart ausgebildet ist, daß sich beim Auseinanderziehen der Aufreißlaschen (35, 36) Reißlinien (43, 44) ergeben, die im wesentlichen parallel zu den Stirnseiten der Packung verlaufen, wobei längs der Stirnseiten dieses Folienteils (12) Streifen stehenbleiben, die zwei einander gegenüberliegende Ränder der Faltkarte (14) überdecken.

2. Packung nach Anspruch 1, dadurch gekennzeichnet, daß die Faltkarte (14) mindestens zwei übereinandergefaltete Flächen (15, 16, 17), zwischen denen sich die wesentliche Menge des Nahtmaterials (45) befindet, und eine Öffnung (20) aufweist, durch die hindurch das Nahtmaterial (45) herausziehbar ist.

3. Packung nach Anspruch 2, dadurch gekennzeichnet, daß die Öffnung (20) sich an der den Aufreißlaschen (35, 36) zugewandten Faltkante (18) der Faltkarte (14) befindet.

4. Packung nach Anspruch 3, dadurch gekennzeichnet, daß die Faltkarte (14) über eine flexible Lasche (23) mit dem einen Folienteil (12) verbunden ist.

5. Packung nach Anspruch 4, dadurch gekennzeichnet, daß die Lasche (23) mehrere querverlaufende Faltlinien (28, 29, 30) mit einander abwechselnden Faltrichtungen aufweist.

6. Packung nach Anspruch 5, dadurch gekennzeichnet, daß mindestens eine der Faltlinien (28, 29, 30) als perforierte Abreißlinie ausgebildet ist.

7. Packung nach Anspruch 5, dadurch gekennzeichnet, daß die Lasche (23) einen eine Faltlinie (30) kreuzenden Schlitz (47) zum Einklemmen des Endes des Nahtmaterials oder einer an dem Ende des Nahtmaterials (35) vorgesehenen Nadel (46) aufweist.

8. Packung nach einem der Ansprüche 4 bis 7, dadurch gekennzeichnet, daß die Faltkarte (14) in der Nähe ihrer der Lasche (23) zugewandten Faltkante (19) Einsteckschlitze (31, 32) für zwei Ecken (33, 34) der gefalteten Lasche (23) aufweist.

9. Packung nach einem der Ansprüche 4 bis 8, dadurch gekennzeichnet, daß die Faltkarte (14) drei im wesentlichen gleich große, entlang ihrer Längskanten durch Faltlinien (18, 19) verbundene Flächen (15, 16, 17) aufweist, von denen an der freien Längskante einer der äußeren Flächen (15) die Lasche (23) angeordnet ist.

10. Packung nach einem der Ansprüche 1 bis 9, dadurch gekennzeichnet, daß das zweite Folienteil ein relativ steifer wannenförmiger Behälter (11) ist, der mit dem ersten Folienteil (12) als Deckel entlang der gegeneinanderliegenden Randbereiche verschlossen ist.

11. Packung nach einem der vorhergehenden Ansprüche, dadurch gekennzeichnet, daß in der Nähe der Aufreißlaschen (35, 36) eine die Folienteile (11, 12) verbindende Schweißnaht (38) in Form zweier nebeneinanderliegender, nach innen weisender Bögen (38) verläuft, die eine gemeinsame Spitze (40) für den Beginn des Aufreißvorganges bilden.

12. Packung nach einem der vorhergehenden Ansprüche, dadurch gekennzeichnet, daß die Aufreißlasche (36) des ersten Folienteiles (12) an ihren seitlichen Fußpunkten Schlitze (41, 42) aufweist, die den Beginn der Aufreißlinien (43, 44) markieren.

## Claims

1. Package for surgical suture material comprising two pieces of film which are positioned against each other at their edges and are joined in a sealing manner, enclosing the suture material and which have tearopen flaps on one edge, and a folding card positioned between the pieces of film and containing the substantial quantity of the suture material from which card projects a thread end of the suture material, characterized in that the one piece of film (12) is so designed that when the tear-open flaps (35, 36) are drawn apart, tear lines (43, 44) are produced which extend substantially in parallel to the end sides of the packagage, while, along the end sides of said piece of film (12) strips remain to cover two opposite edges of the folding card (14).

2. Package according to claim 1, characterized in that the folding card (14) has at least two surfaces (15, 16, 17) folded one over the other and between which the substantial quantity of the suture material (45) is positioned, said card having an aperture (20) through which the suture material (45) can be drawn out.

3. Package according to claim 2, characterized in that the aperture (20) is located at the folding edge (18) of the folding card (14) facing the tear-open flaps (35, 36).

4. Package according to claim 3, characterized in that the folding card (14) is joined to one piece of film (12) via a flexible flap (23).

5. Package according to claim 4, characterized in that the flap (23) has a plurality of transversely extending folding lines (28, 29, 30) having alternating folding directions.

6. Package according to claim 5 characterized in that at least one of the folding lines (28, 29, 30) is designed as a perforated tear line.

7. Package according to claim 5, characterized in that the flap (23) contains a slit (47) crossing a folding line (30) to hold the end of the suture material or a needle (46) provided at the end of the suture material (35).

8. Package according to any of claims 4 to 7, characterized in that in the vicinity of its folding edge (19) facing the flap (23), the folding card (14) contains insertion slits (31, 32) for two corners (33, 34) of the folded flap (23).

9. Package according to any of claims 4 to 8, characterized in that the folding card (14) contains three surfaces (15, 16, 17) of a substantially equal size and joined by folding lines (18, 19) along their longitudinal edges, the flap (23) being positioned on the free longitudinal edge of one of the outer surfaces (15).

10. Package according to any of claims 1 to 9, characterized in that the second piece of film is a relatively rigid trough-shaped container (11) which is closed with the first piece of film (12) as a cover along the peripheral regions which lie against each other.

11. Package according to any of the preceding claims, characterized in that in the vicinity of the tear-open flaps (35, 36), there extends a sealing seam (38) joining the pieces of film (11, 12) and having the shape of two adjacent inwardly pointing arcs (38) which form a common point (40) for the start of the tearing procedure.

12. Package according to any of the preceding claims, characterized in that the tear-open flap (36) of the first piece of film (12) contains slits (41, 42) at the feet of its sides to mark the start of the tear-open lines (43, 44).

**Revendications**

1. Emballage pour fil de suture chirurgicale, comportant deux parties de feuille (11, 12) entourant le fil de suture, appliquées l'une contre l'autre et reliées l'une avec l'autre de façon étanche sur les bords et qui comportent sur un bord des languettes de déchirage ainsi qu'une carte pliée (14) disposée entre les parties de feuille, contenant la quantité principale de matière de suture et de laquelle dépasse une extrémité de fil de suture, caractérisé en ce qu'une des parties de feuille (12) est agencée de manière que, lors de l'écartement mutuel par traction de languettes de déchirement (35, 36) il se forme des lignes de déchirement (43, 44) qui sont orientées essentiellement parallèlement aux côtés frontaux de l'emballage de sorte qu'il subsiste le long des côtés frontaux de cette partie de feuille (12) des bandes qui recouvrent deux bords mutuellement adjacents de la carte pliée (14).

2. Emballage selon la revendication 1, caractérisé en ce que la carte pliée (14) comporte au moins deux surfaces (15, 16, 17) pliées l'une sur l'autre et entre lesquelles se trouve la quantité principale de fil de suture (45) ainsi qu'une ouverture (20) au travers de laquelle peut être sorti le fil de suture (45).

3. Emballage selon la revendication 2, caractérisé en ce que l'ouverture (20) se trouve sur le bord de pliage (18) de la carte (14) qui est tourné vers les languettes de déchirement (35, 36).

4. Emballage selon la revendication 3, caractérisé en ce que la carte pliée (14) est reliée par l'intermédiaire d'une patte flexible (23) à l'une des parties de feuille (12).

5. Emballage selon la revendication 4, caractérisé en ce que la patte (23) comporte plusieurs lignes de pliage (28, 29, 30) orientées transversalement et ayant des directions de pliage successivement alternées.

6. Emballage selon la revendication 5, caractérisé en ce qu'au moins une des lignes de pliage (28, 29, 30) est agencée sous forme d'une ligne de déchirage perforée.

7. Emballage selon la revendication 5, caractérisé en ce que la patte (23) comporte une fente (47) croisant une ligne de pliage (30) et servant à bloquer l'extrémité du fil de suture ou bien une aiguille (46) prévue à l'extrémité de la matière de suture (35).

8. Emballage selon l'une des revendications 4 à 7, caractérisé en ce que la carte pliée (14) comporte, au voisinage de son bord de pliage (19) tourné vers la patte (23) des fentes d'engagement (31, 32) pour deux coins (33, 34) de la patte pliée (23).

9. Emballage selon l'une des revendications 4 à 8, caractérisé en ce que la carte pliée (14) comporte trois surfaces (15, 16, 17) de grandeurs essentiellement égales, reliées le long de leurs bords longitudinaux par des lignes de pliage (18, 19) et en ce que la patte (23) est disposée sur le bord longitudinal libre d'une desdites surfaces qui sont placées à l'extérieur (15).

10. Emballage selon l'une des revendications 1 à 9, caractérisé en ce que la seconde partie de feuille est un récipient en forme de cuvette (11) relativement rigide qui est obturé le long des zones marginales mutuellement opposées par la première partie de feuille (12) servant de couvercle.

11. Emballage selon l'une des revendications précédentes, caractérisé en ce qu'il est prévu au voisinage des languettes de déchirement (35, 36) un joint de soudure (38) reliant les parties de feuille (11, 12) et se présentant sous la forme de deux arcs (38) placés l'un à côté de l'autre, dirigés vers l'intérieur et qui forment une pointe commune (40) pour le début du processus de déchirement.

12. Emballage selon l'une des revendications précédentes, caractérisé en ce que la languette de déchirement (36) de la première partie de feuille (12) comporte, en ses points de base latéraux, des entailles (41, 42) qui marquent le début des lignes de déchirement (43, 44).

FIG. 1

II

10

44

38

42

36

43

40

35

12 41 11

FIG. 2

24 28 23 12 46 30 14 45 11'

36

38 13 29 11

39

35

13

FIG. 3

12

36

24 23

14 45 13

13 35

37 38 11' 11

39

1

FIG. 4

FIG. 5

2